# FASCICULE DE BREVET EUROPEEN

(11) **EP 2 956 436 B1**
(45) Date de publication et mention de la délivrance du brevet: **12.04.2017**
(21) Numéro de dépôt: 14703377.3
(22) Date de dépôt: 10.02.2014
(51) Int. Cl.: C07C 213/06, C07C 303/40, C07C 311/48, C07D 233/60, H01M 10/052, H01M 10/0569, H01M 4/587

(54) **PROCÉDÉ DE SYNTHÈSE DE LIQUIDES IONIQUES À GROUPE FONCTIONNEL CARBONATE ET LIQUIDES IONIQUES AINSI OBTENUS**
VERFAHREN ZUR SYNTHESE VON IONISCHEN FLÜSSIGKEITEN MIT EINER CARBONAT-FUNKTIONSGRUPPE UND SO ERHALTENE IONISCHE FLÜSSIGKEITEN
PROCESS FOR THE SYNTHESIS OF IONIC LIQUIDS WITH A CARBONATE FUNCTIONAL GROUP AND IONIC LIQUIDS THUS OBTAINED

(30) Priorité: 13.02.2013 FR 1351205
(43) Date de publication de la demande: 23.12.2015
(73) Titulaire: Commissariat à l'Énergie Atomique et aux Énergies Alternatives, 75015 Paris (FR); CNRS (Centre National de la Recherche Scientifique), 75016 Paris Cedex 16 (FR); CPE Lyon, 69616 Villeurbanne Cedex (FR); Université Claude Bernard Lyon 1, 69622 Villeurbanne, Cedex (FR)
(72) Inventeur: SROUR, Hassan, F-69100 Villeurbanne (FR); ROUAULT, Hélène, F-38420 Le Versoud (FR); SANTINI, Catherine, F-69660 Collonges Mont D'or (FR)
(74) Mandataire: Brizio Delaporte, Allison
(86) Numéro de dépôt international: PCT/EP2014/052518
(87) Numéro de publication internationale: WO 2014/124892

(56) Documents cités:
- EP-A1- 1 548 866
- EP-A1- 2 450 999
- JP-B- 4 478 790
- D.Q. NGUYEN ET AL: "Synthesis and characterization of quaternary ammonium-based ionic liquids containing an alkyl carbonate group", BULLETIN OF THE KOREAN CHEMICAL SOCIETY, vol. 28, no. 12, 1 décembre 2007 (2007-12-01), pages 2299-2302, XP009117677, ISSN: 0253-2964 cité dans la demande

## Description

La présente invention concerne un procédé de synthèse de liquides ioniques à groupe fonctionnel carbonate et les liquides ioniques ainsi obtenus.

Les liquides ioniques sont des sels ayant une température de fusion inférieure à 100°C.

Les liquides ioniques sont composés de cations organiques et d'anions (in)organiques. Ils présentent de nombreux avantages et ont connus ces dernières années un engouement particulier dans divers domaines de la chimie dont les solvants verts, la catalyse ou bien encore la séparation.

Les liquides ioniques obtenus par le présent procédé peuvent trouver leurs applications dans la catalyse enzymatique ou dans la dissolution de la biomasse.

Les liquides ioniques peuvent également être utilisés comme électrolytes dans des batteries au lithium. Ils sont considérés comme plus sûrs du fait de leur faible volatilité et inflammabilité.

En parallèle, il a été développé des électrodes en graphite pour les batteries à base de lithium. Ces électrodes ont l'avantage d'être fabriquées à faible coût et d'être plus écologiques que les électrodes de l'art antérieur.

Toutefois, les liquides ioniques connus à ce jour ne peuvent être utilisés avec ces électrodes en graphite notamment car les ions lithium de la batterie ne peuvent s'intercaler entre les feuillets de graphite. Des additifs tels que le vinylène de carbonate (VC) sont donc ajoutés pour former une interface d'électrolyte solide (SEI en anglais Solide Electrolyte Interface) sur l'électrode en graphite permettant ainsi de prolonger la durée de vie des batteries. Or, les additifs nécessaires sont des solvants organiques volatiles chers ce qui limite l'intérêt des liquides ioniques dans le domaine des batteries au lithium.

Une publication, Nguyen, Bull. Korean Chem. Soc. 2007 28, 2299, a présenté des liquides ioniques comprenant un groupe fonctionnel carbonate. Ce type de liquide ionique ne nécessite donc plus d'ajout d'additif pour être utilisé dans les batteries lithium à électrode graphite. La synthèse de ces liquides ioniques fait intervenir une étape de métathèse d'anions impliquant un sel de lithium. Les liquides ioniques obtenus ne présentent cependant pas les propriétés satisfaisantes pour être utilisés comme électrolyte dans des batteries. Les auteurs ont identifié, par spectroscopie à infrarouge, une interaction entre la fonction carbonate et le sel de lithium. Cette interaction implique la présence au niveau du liquide ionique final de lithium.

Le document brevet JP4478790B2 divulgue un procédé d'obtention de liquides ioniques. Le procédé implique une étape comprenant l'ajout d'un sel de lithium. Cette interaction implique également la présence au niveau du liquide ionique final de lithium.

Par conséquent, ces liquides ioniques ne sont pas utilisables comme électrolytes dans les batteries lithium. Il existe donc le besoin de proposer un procédé de synthèse de liquide ionique à fonction carbonate résolvant tout ou partie des inconvénients ci-dessus mentionnés.

A cet effet, la présente invention concerne un procédé de synthèse de liquides ioniques comprenant un groupe fonctionnel carbonate, le procédé comprend une étape de réaction A sans ajout de lithium entre un premier réactant choisi parmi un imidazolium, un pyrrolidinium ou un ammonium et un deuxième réactant étant un méthyl formate imidazolium.

La réaction de synthèse du liquide ionique selon l'invention ne met pas en jeu de lithium ce qui permet d'obtenir un liquide ionique pur comprenant un groupement fonctionnel carbonate. Le liquide ionique obtenu est donc directement utilisable comme électrolyte notamment dans des batteries au lithium ayant au moins une électrode en graphite.

Le liquide ionique à fonction carbonate synthétisé ne comprend pas de lithium. Il ne comporte pas non plus d'halogénure.

Selon un mode de réalisation avantageux, le procédé selon l'invention permet de recycler le produit secondaire obtenu lors de la réaction A de synthèse du liquide ionique. Le produit secondaire est recyclé en réactant pour ladite réaction A. Le procédé ne génère donc pas de déchet devant être traité.

Le présent procédé est donc rapide et peu coûteux à mettre en oeuvre.

D'autres buts et avantages apparaîtront au cours de la description qui suit qui présente un mode de réalisation de l'invention illustratif mais non limitatif.

Suivant des variantes préférées mais non limitatives, l'invention est telle que :
- le premier réactant est un alcool d'imidazolium, de pyrrolidinium ou d'ammonium ;
- le premier réactant est un sel d'alcool d'imidazolium, d'alcool de pyrrolidinium ou d'alcool d'ammonium dont l'anion est le bis(trifluoromethanesulfonyl)imide NTf₂; l'étape de réaction A conduit à deux produits dont un produit principal étant un liquide ionique comprenant un groupe fonctionnel carbonate et un produit secondaire étant un méthyl imidazolium ;
- elle comprend une étape ultérieure de recyclage par réaction d'Hoffmann du produit secondaire méthyl imidazolium en un alcool d'imidazolium, formant le premier réactant de la réaction A ;
- le méthyl imidazolium réagit avec le 2-chloro-1-éthanol pour former le 1-éthanol-3-méthyl imidazolium chloride ;
- le chlore du 1-éthanol-3-méthyl imidazolium chloride est substitué par le Ntf2 par réaction avec LiNtf2 ;
- l'étape de réaction A est réalisée à température ambiante dans une solution à base d'acétonitrile sous atmosphère d'argon ; de préférence dans de l'acétonitrile ;
- le deuxième réactant est un chloro-méthyl formate imidazolium ;
- le deuxième réactant est obtenu par réaction entre le 1-méthylimidazole et le méthylchloroformate ;
- l'étape de la réaction est réalisée à 0°C dans une solution à base d'acétonitrile pendant 2h ; de préférence dans de l'acétonitrile ;
- le deuxième réactant obtenu par réaction entre le 1-méthylimidazole et le méthylchloroformate est directement mélangé au premier réactant de la réaction A.

Suivant un autre aspect l'invention concerne un liquide ionique comprenant un groupe fonctionnel carbonate obtenu suivant le procédé de l'invention ci-dessus.

Suivant un autre aspect, l'invention concerne une batterie au lithium et au moins une électrode en graphite comprenant un liquide ionique selon l'invention. Avantageusement, le liquide ionique fait office d'électrolyte dans la batterie.

Un accumulateur au lithium, conformément aux connaissances générales de l'homme du métier, est généralement constitué par :
- deux électrodes, à savoir une électrode positive et une électrode négative. L'électrode positive comprend généralement, en tant que matière électrochimiquement active, des matériaux d'intercalation du lithium tels que des oxydes lamellaires de métaux de transition lithiés, des olivines (LiFePO4) ou des spinelles (par exemple le spinelle LiNi0,5Mn1,5O4). L'électrode négative comprend généralement, en tant que matière électrochimiquement active des matériaux d'intercalation tels que le carbone graphite (Cgr).

- des collecteurs de courant, généralement en cuivre pour l'électrode négative, ou en aluminium pour l'électrode positive, qui permettent la circulation des électrons, et donc la conduction électronique, dans le circuit extérieur,
- un électrolyte liquide ionique comprenant un sel de Lithium où se produit la conduction ionique qui assure le passage des ions lithium d'une électrode à l'autre.

Les dessins ci-joints sont donnés à titre d'exemples et ne sont pas limitatifs de l'invention. Ils représentent seulement un mode de réalisation de l'invention et permettront de la comprendre aisément.

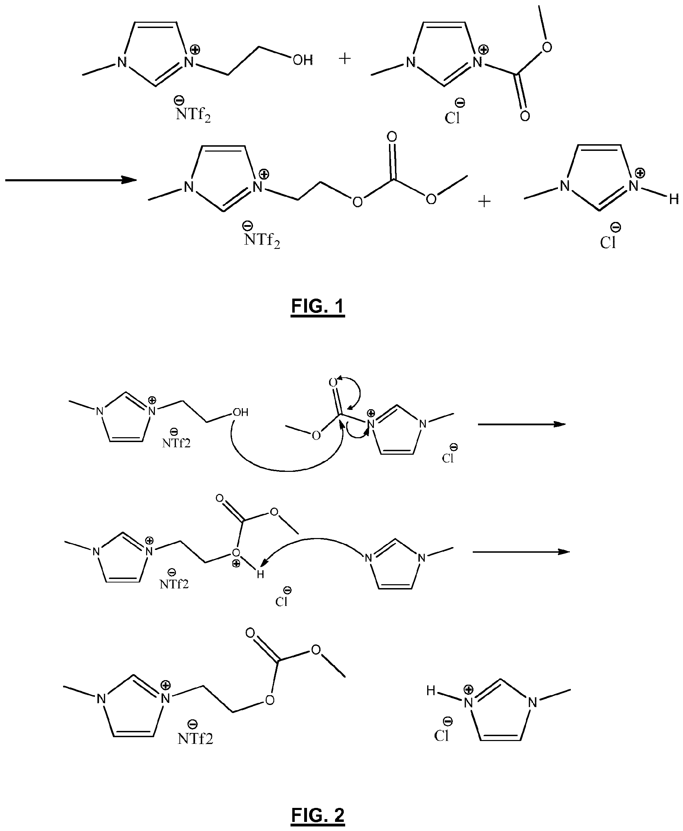

La figure 1 illustre un exemple de l'étape de réaction A du procédé de synthèse selon l'invention.
La figure 2 illustre le mécanisme de la réaction A de synthèse du procédé selon l'invention.

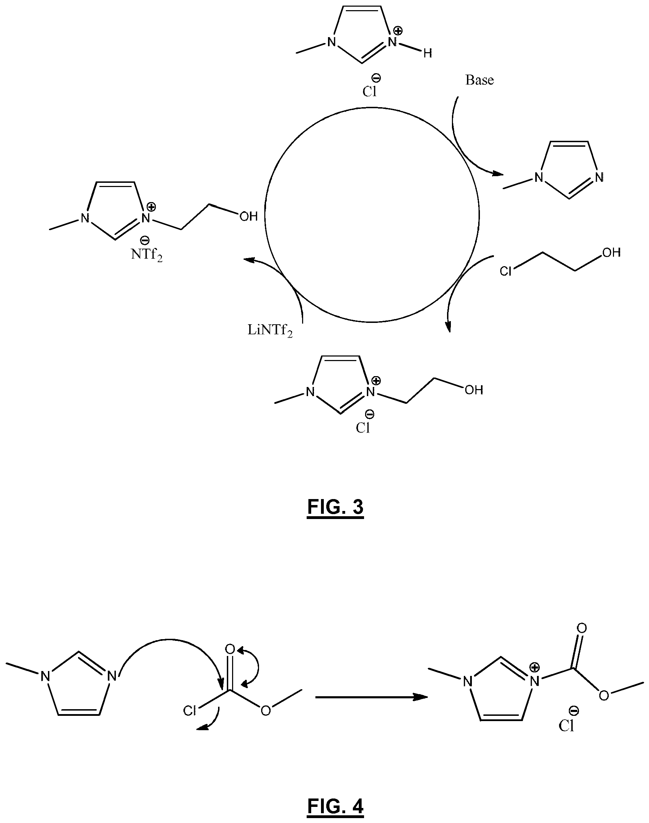

La figure 3 illustre un exemple de l'étape de recyclage du produit secondaire issu de la réaction A du procédé selon l'invention.
La figure 4 illustre le mécanisme de la réaction de la synthèse du deuxième réactant du procédé selon l'invention.

Selon l'invention, le procédé de synthèse d'un liquide ionique comprend une réaction A entre deux réactants 1,2.

Le premier réactant est choisi parmi un imidazolium un pyrrolidinium ou un ammonium selon le type de liquide ionique que l'on souhaite synthétiser. Ce premier réactant 1 est avantageusement, un alcool d'imidazolium, de pyrrolidinium ou d'ammonium. Le premier réactant est sous forme de sel dont l'anion est le bis(trifluorométhanesulfonyl)imide (aussi appelé NTf2 ou TFSI), et dont le cation est un imidazolium, ou un pyrrolidinium ou un ammonium. L'anion NTf2 confère au liquide ionique un caractère hydrophobe permettant une synthèse et purification plus aisées.

A titre d'exemple non limitatif, le premier réactant 1 est du 1-éthanol-3-méthylimidazolium bis(trifluorométhanesulfonyl)imide aussi appelé C1C2OHlm-NTf2. Le premier réactant peut être obtenu directement dans le commerce.

Le deuxième réactant est un méthyl formate imidazolium. Préférentiellement, le deuxième réactant est un chloro-méthyl formate imidazolium. A titre d'exemple, le deuxième réactant 2 est un 1-méthyl-3-méthylformatelmidazolium chloride. Le deuxième réactant est avantageusement obtenu par une étape de réaction entre le méthyl imidazole et le méthylchloroformate. La réaction se déroule avantageusement à 0°C dans de l'acétonitrile. La réaction a une durée de l'ordre de 2h.

Préférentiellement, une fois le deuxième réactant synthétisé, le premier réactant est directement mélangé au deuxième réactant sans séparation de ce dernier.

Ceci facilite grandement le procédé de synthèse selon l'invention.

L'étape de réaction A entre le premier réactant et le deuxième réactant permet la synthèse directe du liquide ionique portant au moins une fonction carbonate. Cette étape de réaction A est avantageusement réalisée dans de l'acétonitrile à température ambiante, plus précisément entre 18°C et 25°C sous atmosphère d'argon.

A titre d'exemple, les liquides ioniques pouvant êtres obtenus par le procédé selon l'invention sont, pour le cation :
1-[2-(methoxycarbonyloxy)ethyl]-3-methylimidazolium
1-[2-(ethoxycarbonyloxy)ethyl]-3-methylimidazolium
1-[2-(propoxycarbonyloxy)ethyl]-3-methylimidazolium
1-[2-(isopropoxycarbonyloxy)ethyl]-3-methylimidazolium
N-[2-(methoxycarbonyloxy)ethyl]-N'-methylpyrrolidinium
N-[2-(ethoxycarbonyloxy)ethyl]-N'-methylpyrrolidinium
N-[2-(propoxycarbonyloxy)ethyl]-N'-methylpyrrolidinium
N-[2-(isopropoxycarbonyloxy)ethyl]-N'-methylpyrrolidinium
N-trimethyl-N-2-[(methoxycarbonyloxy)ethyl]ammonium
N-trimethyl-N-2-[(ethoxycarbonyloxy)ethyl]ammonium
N-trimethyl-N-2-[(propoxycarbonyloxy)ethyl]ammonium
N-trimethyl-N-2-[(isopropoxycarbonyloxy)ethyl]ammonium;

L'anion associé étant le bis(trifluorométhanesulfonyl)imide.

L'étape de réaction A conduit à deux produits dont un produit principal étant le liquide ionique comprenant au moins une fonction carbonate et un produit secondaire.

Le liquide ionique obtenu comporte une fonction carbonate. Avantageusement, il est exempt d'halogénure. En effet, le chlore du deuxième réactant est l'anion du produit secondaire. En outre, l'étape de réaction A n'implique pas de réactant comportant du lithium. Le procédé de synthèse selon l'invention est réalisé sans lithium. Il n'y a pas coordination avec d'halogénure ou de lithium avec la fonction carbonate.

Le produit secondaire comporte le chloro-imidazolium du deuxième réactant. A titre d'exemple, le produit secondaire est un méthyl Imidazolium chloride.

Le procédé de l'invention présente également l'avantage de permettre le recyclage du produit secondaire en un réactant plus précisément en premier réactant. Le recyclage du produit secondaire de l'étape de réaction A se fait par réaction d'Hoffman. Ce cycle de réaction est décrit dans la demande de brevet WO 01/77081. L'étape de recyclage est préférentiellement effectuée entre le produit secondaire et le 2-chloro-1-éthanol. Le produit de cette réaction comporte un halogénure qui est avantageusement substitué par un anion choisi pour le premier réactant. Cet anion est coordonné au lithium. A titre d'exemple, le chlore est remplacé par le NTf2 par un échange d'anion avec le lithium-NTf2 (LiNTf2). La fonction alcool portée par le premier réactant et également par le sel d'halogénure dont l'halogénure est remplacé par le NTf2 ne permet pas de chélation du cation de lithium. Le produit obtenu est un premier réactant pour la réaction A de synthèse du liquide ionique. Le produit obtenu présente une pureté de 99,9%.

Le procédé selon l'invention n'utilise pas de lithium et permet avantageusement le recyclage de produit secondaire. Il présente ainsi de nombreux avantages pour être utilisé dans la chimie verte.

### Exemple 1 : Synthèse du deuxième réactant : 1-méthyl-3-méthylformatelmidazolium chloride

Du méthyl chloroformate (52g, 550mmol) est ajouté goutte à goutte à une solution de 1-méthylimidazole (41g, 500mmol) dans de l'acétonitrile (200mL) à 0°C et la réaction dure 2h. Le 1-méthyl-3-méthylformatelmidazolium chloride est obtenu.

### Exemple 2 : Synthèse du liquide ionique comprenant une fonction carbonate : 1-[2-(methoxycarbonyloxy)ethyl]-3-methylimidazolium bis(trifluorométhanesulfonyl)imide [EMCMIm]TFSI.

Le premier réactant, 1-ethanol-3-methylimidazolium bis(trifluoromethanesulfonyl)imide, (50g, 122mmol) et le deuxième réactant, tel qu'obtenu à l'exemple 1 : 1-méthyl-3-méthylformatelmidazolium chloride (15,74g, 111mmol) sont mis en contact à température ambiante pendant une journée. Un produit légèrement jaune est obtenu. Le liquide ionique à fonction carbonate est obtenu avec un rendement d'environ 90%.

### Exemple 3 : Recyclage du produit secondaire en premier réactant :

Un exemple de procédé de recyclage est par exemple décrit dans la demande de brevet internationale publiée sous le numéro WO2005068404.

## Revendications

1. Procédé de synthèse de liquides ioniques comprenant un groupe fonctionnel carbonate **caractérisé en ce qu'**il comprend une étape de réaction A, sans ajout de lithium, entre un premier réactant choisi parmi un imidazolium, un pyrrolidinium ou un ammonium et un deuxième réactant étant un méthyl formate imidazolium.

2. Procédé selon la revendication précédente, dans lequel le premier réactant est un alcool d'imidazolium, de pyrrolidinium ou d'ammonium.

3. Procédé selon l'une quelconque des revendications précédentes dans lequel le premier réactant est un sel d'alcool d'imidazolium, d'alcool de pyrrolidinium ou d'alcool d'ammonium dont l'anion est le bis(trifluorométhanesulfonyl)imide (Ntf2).

4. Procédé selon l'une quelconque des revendications précédentes dans lequel l'étape de réaction A conduit à deux produits dont un produit principal étant un liquide ionique comprenant un groupe fonctionnel carbonate et un produit secondaire étant un méthyl imidazolium.

5. Procédé selon la revendication précédente comprenant une étape ultérieure de recyclage par réaction d'Hoffmann du produit secondaire méthyl imidazolium en un alcool d'imidazolium, formant le premier réactant de la réaction A.

6. Procédé selon la revendication précédente dans lequel le méthyl imidazolium réagit avec le 2-chloro-1-éthanol pour former le 1-éthanol-3-méthyl imidazolium chloride.

7. Procédé selon la revendication précédente dans lequel le chlore du 1-éthanol-3-méthyl imidazolium chloride est substitué par le Ntf2 par réaction avec LiNtf2.

8. Procédé selon l'une quelconque des revendications précédentes dans lequel le liquide ionique obtenu à l'issu de l'étape de réaction A est
1-[2-(methoxycarbonyloxy)ethyl]-3-methylimidazolium
1-[2-(ethoxycarbonyloxy)ethyl]-3-methylimidazolium
1-[2-(propoxycarbonyloxy)ethyl]-3-methylimidazolium
1-[2-(isopropoxycarbonyloxy)ethyl]-3-methylimidazolium
N-[2-(methoxycarbonyloxy)ethyl]-N'-methylpyrrolidinium
N-[2-(ethoxycarbonyloxy)ethyl]-N'-methylpyrrolidinium
N-[2-(propoxycarbonyloxy)ethyl]- N'-methylpyrrolidinium
N-[2-(isopropoxycarbonyloxy)ethyl]-N'-methylpyrrolidinium
N-trimethyl-N-2-[(methoxycarbonyloxy)ethyl]ammonium
N-trimethyl-N-2-[(ethoxycarbonyloxy)ethyl]ammonium
N-trimethyl-N-2-[(propoxycarbonyloxy)ethyl]ammonium
N-trimethyl-N-2-[(isopropoxycarbonyloxy)ethyl]ammonium
chacun asocié à l'anion bis(trifluorométhanesulfonyl)imide.

9. Procédé selon l'une quelconque des revendications précédentes dans lequel l'étape de réaction A est réalisée à température ambiante dans une solution à base d'acétonitrile sous atmosphère d'argon.

10. Procédé selon l'une quelconque des revendications précédentes dans lequel le deuxième réactant est un chloro-méthyl formate imidazolium.

11. Procédé selon la revendication précédente dans lequel le deuxième réactant est obtenu par réaction entre le 1-méthylimidazole et le méthylchloroformate.

12. Procédé selon la revendication précédente dans lequel la réaction est réalisée à 0°C dans une solution à base d'acétonitrile pendant 2h.

13. Procédé selon l'une quelconque des deux revendications précédentes dans lequel le deuxième réactant obtenu par réaction entre le 1-méthylimidazole et le méthylchloroformate est directement mélangé au premier réactant de la réaction A.

14. Liquide ionique comprenant un groupe fonctionnel carbonate obtenu suivant le procédé selon l'une quelconque des revendications précédentes et étant exempt de lithium.

15. Batterie comprenant du lithium et au moins une électrode en graphite comprenant un liquide ionique selon la revendication précédente faisant office d'électrolyte.

## Patentansprüche

1. Verfahren zur Synthese von ionischen Flüssigkeiten, eine Carbonat-Funktionsgruppe umfassend, **dadurch gekennzeichnet, dass** es einen Reaktionsschritt A, ohne Lithium hinzuzufügen, zwischen einem ersten Reaktionspartner, der unter einem Imidazol, einem Pyrrolidinium oder einem Ammonium ausgewählt wird, und einem zweiten Reaktionspartner umfasst, der aus einem Methylformiatimidazol besteht.

2. Verfahren nach dem vorhergehenden Anspruch, wobei es sich bei dem ersten Reaktionspartner um einen Imidazol-, Pyrrolidinium- oder Ammoniumalkohol handelt.

3. Verfahren nach irgendeinem der vorhergehenden Ansprüche, wobei es sich bei dem ersten Reaktionspartner um ein Salz des Imidazolalkohols, des Pyrrolidiniumalkohols oder des Ammoniumalkohols handelt, dessen Anion das Bis(trifluormethansulfonyl)imid (Ntf2) ist.

4. Verfahren nach irgendeinem der vorhergehenden Ansprüche, wobei der Reaktionsschritt A zu zwei Produkten führt, wobei es sich bei einem Hauptprodukt um eine ionische Flüssigkeit handelt, die eine Carbonat-Funktionsgruppe umfasst, und es sich bei einem sekundären Produkt um ein Methylimidazol handelt.

5. Verfahren nach dem vorhergehenden Anspruch, einen letzten Recyclingschritt durch Hoffmann-Reaktion des sekundären Produktes Methylimidazol in einen Imidazoalkohol umfassend, wodurch der erste Reaktionspartner der Reaktion A gebildet wird.

6. Verfahren nach dem vorhergehenden Anspruch, wobei das Methylimidazol mit dem 2-Chlor-1-Ethanol reagiert, um das 1-Ethanol-3-Methylimidazolchlorid zu bilden.

7. Verfahren nach dem vorhergehenden Anspruch, wobei das Chlor des 1-Ethanol-3-Methylimidazolchlorid durch das Ntf2 durch Reaktion mit LiNtf2 ersetzt wird.

8. Verfahren nach irgendeinem der vorhergehenden Ansprüche, wobei es sich bei der ionischen Flüssigkeit, die am Ende des Rektionsschrittes A erhalten wird, um
1-[2-(Methoxycarbonyloxy)ethyl]-3-methylimidazol
1-[2-(Ethoxycarbonyloxy)ethyl]-3-methylimidazol
1-[2-(Propoxycarbonyloxy)ethyl]-3-methylimidazol
1-[2-(Isopropoxycarbonyloxy)ethyl]-3-methylimidazol
N-[2-(Methoxycarbonyloxy)ethyl]-N'-methylpyrrolidinium
N-[2-(Ethoxycarbonyloxy)ethyl]- N'-methylpyrrolidinium
N-[2-(Propoxycarbonyloxy)ethyl]- N'- methylpyrrolidinium
N-[2-(Isopropoxycarbonyloxy)ethyl]-N'-methylpyrrolidinium
N-Trimethyl-N-2-[(methoxycarbonyloxy)ethyl]ammonium
N-Trimethyl-N-2-[(ethoxycarbonyloxy)ethyl]ammonium
N-Trimethyl-N-2-[(propoxycarbonyloxy)ethyl]ammonium
N-Trimethyl-N-2-[(isopropoxycarbonyloxy)ethyl]ammonium
handelt, die jeweils dem Anion Bis(trifluoromethansulfonyl)imid zugeordnet sind.

9. Verfahren nach irgendeinem der vorhergehenden Ansprüche, wobei der Reaktionsschritt A bei Umgebungstemperatur in einer Lösung auf der Basis von Acetonitril in Argonatmosphäre erfolgt.

10. Verfahren nach irgendeinem der vorhergehenden Ansprüche, wobei es sich bei dem zweiten Reaktionspartner um ein Chlor-Methylformiatimidazol handelt.

11. Verfahren nach dem vorhergehenden Anspruch, wobei der zweite Reaktionspartner durch Reaktion zwischen dem 1-Methylimidazol und dem Methylchlorformiat erhalten wird.

12. Verfahren nach dem vorhergehenden Anspruch, wobei die Reaktion bei 0° in einer Lösung auf Acetonitrilbasis während 2 Stunden erfolgt.

13. Verfahren nach irgendeinem der beiden vorhergehenden Ansprüche, wobei der zweite Reaktionspartner, der durch Reaktion zwischen dem 1-Methylimidazol und dem Methylchlorformiat erhalten wird, direkt mit dem ersten Reaktionspartner der Reaktion A gemischt wird.

14. Ionische Flüssigkeit, eine Carbonat-Funktionsgruppe umfassend, die gemäß dem Verfahren nach irgendeinem der vorhergehenden Ansprüche erhalten wird, und die frei von Lithium ist.

15. Batterie, die Lithium und mindestens eine Graphitelektrode umfasst, die eine ionische Flüssigkeit nach dem vorhergehenden Anspruch umfasst, und als Elektrolyt fungiert.

## Claims

1. A method for synthesizing ionic liquids comprising a carbonate functional group **characterized in that** it comprises a step of reaction A without addition of lithium between a first reactant selected among an imidazolium, a pyrrolidinium or an ammonium and a second reactant being a methyl formate imidazolium.

2. A method according to the preceding claim, wherein the first reactant is an imidazolium, a pyrrolidinium or an ammonium alcohol.

3. A method according to any one of the preceding claims, wherein the first reactant is an imidazolium alcohol, a pyrrolidinium alcohol or an ammonium alcohol salt, the anion of which is bis(trifluorométhanesulfonyl)imide (NTf2).

4. A method according to any one of the preceding claims, wherein the step of reaction A results in two products, with a main product being an ionic liquid comprising a carbonate functional group and a secondary product being a methyl imidazolium.

5. A method according to the preceding claim, comprising a further step of recycling, by Hoffmann reaction, the secondary product methyl imidazolium into an imidazolium alcohol, forming the first reactant of the reaction A.

6. A method according to the preceding claim, wherein methyl imidazolium reacts with 2-chloro-1-ethanol to form 1-ethanol-3-methyl imidazolium chloride.

7. A method according to the preceding claim, wherein the chlorine of 1-ethanol-3-methyl imidazolium chloride is substituted by NTf2, by reaction with LiNtf2.

8. A method according to any one of the preceding claims, wherein the ionic liquid obtained upon completion of the step of reaction A is
1-[2-(methoxycarbonyloxy)ethyl]-3-methylimidazolium
1-[2-(ethoxycarbonyloxy)ethyl]-3-methylimidazolium
1-[2-(propoxycarbonyloxy)ethyl]-3-methylimidazolium
1-[2-(isopropoxycarbonyloxy)ethyl]-3-methylimidazolium
N-[2-(methoxycarbonyloxy)ethyl]-N'-methylpyrrolidinium
N-[2-(ethoxycarbonyloxy)ethyl]-N'-methylpyrrolidinium
N-[2-(propoxycarbonyloxy)ethyl]-N'-methylpyrrolidinium
N-[2-(isopropoxycarbonyloxy)ethyl]-N'-methylpyrrolidinium
N-trimethyl-N-2-[(methoxycarbonyloxy)ethyl]ammonium
N-trimethyl-N-2-[(ethoxycarbonyloxy)ethyl]ammonium
N-trimethyl-N-2-[(propoxycarbonyloxy)ethyl]ammonium
N-trimethyl-N-2-[(isopropoxycarbonyloxy)ethyl]ammonium
each one being associated with the bis(trifluoromethanesulfonyl)imide anion.

9. A method according to any one of the preceding claims, wherein the step of reaction A is executed at ambient temperature in an acetonitrile-based solution in argon atmosphere.

10. A method of any one of the preceding claims, wherein the second reactant is a chloromethyl formate imidazolium.

11. A method according to the preceding claim, wherein the second reactant is obtained by a reaction between 1-methylimidazole and methylchloroformate.

12. A method according to the preceding claim, wherein the reaction is executed at 0°C in an acetonitrile-based solution for 2 hours.

13. A method according to one of the preceding two claims, wherein the second reactant obtained by a reaction between 1-methylimidazole and methylchloroformate is directly mixed with the first reactant of reaction A.

14. An ionic liquid comprising a carbonate functional group obtained according to the method according to any one of the preceding claims and being lithium-free.

15. A battery comprising lithium and at least one graphite electrode comprising an ionic liquid according to the preceding claim, which is used as an electrolyte.
